# EUROPÄISCHE PATENTANMELDUNG

(11) **EP 0 543 081 A1**
(43) Veröffentlichungstag der Anmeldung: **26.05.1993**
(21) Anmeldenummer: 92110944.3
(22) Anmeldetag: 29.06.1992
(51) Int. Cl.: G01N 27/411

(54) **Tauchsensor für Metallschmelzen**

(30) Priorität: 28.10.1991 DE 4135510
(71) Anmelder: Heraeus Electro-Nite International N.V., B-2000 Antwerpen (BE)
(72) Erfinder: Verstreken, Paul Clement, B-3110 Rotselaar-Heikant (BE); Neyens, Guido Jacobus, B-3680 Opoeteren (BE)

(57) **Zusammenfassung**

Die Erfindung betrifft einen Tauchsensor für Metallschmelzen, der einen elektrisch leitenden Trägerstift (1) aufweist, dessen eines Ende in einem feuerfesten Halterungs-Werkstoff (2) gehaltert ist und dessen anderes, freies Ende einen ersten Überzug aus einem Referenz-Material (3) und darauf einen zweiten Überzug aus einem Festelektrolyt-Material (6) aufweist, wobei der Trägerstift (1) im Bereich seines gehalterten Endes einen dritten Überzug aus einem feuerfesten Werkstoff (4) aufweist, der einerseits in den Halterungs-Werkstoff (2) hineinreicht und der sich andererseits bis zum Festelektrolyt-Material erstreckt. Um eine kurze Ansprechzeit und einen stabilen EMK-Meßwert zu erreichen, überdeckt das Festelektrolyt-Material (6) mindestens den Endbereich des ihm benachbarten feuerfesten Werkstoffes (4). Die Erfindung betrifft ebenfalls ein Verfahren zur Herstellung eines Zwischenproduktes eines Tauchsensors; wonach die verschiedenen Schichtwerkstoffe des Tauchsensors in vorgegebener Reihenfolge aufgebraucht werden.

## Beschreibung

Die Erfindung betrifft einen Tauchsensor für Metallschmelzen, der einen elektrisch leitenden Trägerstift aufweist, dessen eines Ende in einem fernerfesten Halterungs-Werkstoff gehaltert ist und dessen anderes, freies Ende einen ersten Überzug aus einem Referenz-Material und darauf einen zweiten Überzug aus einem Festelektrolyt-Material aufweist, wobei der Trägerstift im Bereich seines gehalterten Endes einen dritten Überzug aus einem feuerfesten Werkstoff aufweist, der einerseits in den Halterungs-Werkstoff hineinreicht und der sich andererseits bis zum Festelektrolyt-Material erstreckt. Die Erfindung betrifft weiterhin ein Verfahren zur Herstellung eines Zwischenproduktes eines Tauchsensors.

Derartige Tauchsensoren sind aus der JP-A1 61-60154 bekannt. Die dort beschriebenen Tauchsensoren weisen einen Metalldraht als Trägerstift auf, der an seinem einen Ende in einen Halterungs-Werkstoff eingebettet ist. An seinem anderen Ende ist der Trägerstift weiterhin mit einem Referenz-Material aus einem Metall-Metalloxid überzogen. Auf diesem Referenz-Material ist eine Schicht aus einem Festelektrolyt-Material angeordnet. Im Bereich seines gehalterten Endes ist der Trägerstift mit einem feuerfesten Werkstoff überzogen, der einerseits in den Halterungs-Werkstoff hineinreicht und sich andererseits bis zu dem Festelektrolyt-Material erstreckt, wobei das Festelektrolyt-Material teilweise von dem feuerfesten Werkstoff überdeckt werden kann.

Bei einem derartigen Aufbau kann der feuerfeste Werkstoff in der Metallschmelze korrodieren, wobei sich in dem Bereich, in dem der feuerfeste Werkstoff außerhalb des Halterungs-Werkstoffes an dem Festelektrolyt-Werkstoff anliegt, die elektrochemischen Eigenschaften des Tauchsensors ändern. Dadurch unterliegt der in diesem Bereich vorhandene Teil der zu messenden EMK (Elektromotorische Kraft) während des Meßvorganges starken Änderungen, so daß sich ein konstanter EMK-Wert erst nach einer langen Tauchzeit oder überhaupt nicht einstellt. Bei einer Überlappung des Festelektrolyt-Materials durch den feuerfesten Werkstoff wird eine zusätzliche EMK, die bis in einen Bereich unterhalb des feuerfesten Werkstoffes hinein entsteht, gemessen. Dieser Bereich ist undefiniert und führt deshalb ebenfalls zu einer Verzerrung der Werte bei Messung der EMK.

Ein ähnlicher Tauchsensor mit dem oben beschriebenen Schichtenaufbau ist aus der JP-A1 60-53053 bekannt. Auch bei ihm treten die vorstehend angegebenen Ungenauigkeiten auf.

Ausgehend von dem vorstehend beschriebenen Stand der Technik liegt der vorliegenden Erfindung die Aufgabe zugrunde, einen Tauchsensor zu schaffen, der nach einer kurzen Tauchzeit einen stabilen EMK-Meßwert bildet. Weiterhin ist es Aufgabe der Erfindung, ein Verfahren zur Herstellung eines Zwischenproduktes für einen solchen Tauchsensors anzugeben.

Die Aufgabe wird für einen Tauchsensor der eingangs beschriebenen Art erfindungsgemäß dadurch gelöst, daß das Festelektrolyt-Material mindestens den Endbereich des ihm benachbarten feuerfesten Werkstoffes überdeckt.

Durch diese Maßnahme wird einerseits die Korrosion des feuerfesten Werkstoffes in einem Bereich verhindert, in dem Teile der zu messenden EMK wirken und es wird der Bereich, in dem die EMK gemessen wird, scharf begrenzt. Die zu messende EMK unterliegt daher keinen Schwankungen infolge Korrosionserscheinungen oder durch undefinierte Meßstellen. Schon nach einer kurzen Tauchzeit, in der sich ein thermisches und elektrochemisches Gleichgewicht zwischen dem Tauchsensor und der ihn umgebenden Metallschmelze einstellt, stellt sich ein konstanter EMK-Wert ein.

Verfahrensgemäß wird die Aufgabe dadurch gelöst, daß auf einem Trägerstift auf dessen einem Ende ein Überzug aus einem Referenz-Material aufgebracht wird, daß danach dieses Ende derart maskiert wird, daß das Referenz-Material, das dem Ende abgewandt ist, unmaskiert verbleibt und auf dem unmaskierten Teil des Trägerstiftes ein Überzug aus feuerfestem Werkstoff aufgebracht wird, und daß anschließend auf das Referenz-Material ein Überzug aus einem Festelektrolyt-Material nach Entfernung der Maskierung so aufgebracht wird, daß dieser Überzug das dem Ende des Trägerstiftes benachbarte Ende des Überzugs aus feuerfestem Werkstoff überdeckt. Mit diesem Verfahren ist es möglich, ein Zwischenprodukt für einen Tauchsensor herzustellen, welcher die Eigenschaften des oben beschriebenen erfindungsgemäßen Tauchsensors für Metallschmelzen aufweist.

Vorzugsweise ist der feuerfeste Werkstoff mit einem gegenüber der Metallschmelze korrosionsbeständigen Material überzogen. Zweckmäßigerweise kann dieses korrosionsbeständige Material ein Festelektrolyt-Werkstoff, insbesondere dasselbe Material wie der Festelektrolyt-Überzug, sein. Dadurch werden die korrosionsbedingten EMK-Schwankungen besonders nachhaltig unterdrückt. In dem Fall, daß das korrosionsbeständige Material dasselbe Material ist wie der Festelektrolyt-Überzug, ist der Tauchsensor sehr einfach herstellbar, da eine geschlossene Schicht nur eines Materials aufzubringen ist.

Vorzugsweise beginnt der feuerfeste Werkstoff in einem Abstand von 1 bis 6 mm, insbesondere in einem Abstand von 1,5 bis 3,5 mm vom freien, also nicht im Halterungs-Werkstoff eingebetteten Ende des Trägerstiftes. Es hat sich gezeigt, daß dadurch die zu messende EMK in einem definierten, relativ kleinen Bereich längs des Tauchsensors entsteht. In diesem relativ kleinen Bereich, der bevorzugt 2,5 mm lang ist, sind Temperaturschwankungen, die durch einen evtl. in der Schmelze durch das Eintauchen des Tauchsensors sich ausbildenden Temperaturgradienten bedingt sind, so klein, daß die EMK, die auch von der Temperatur der Metallschmelze abhängig ist, von Temperaturdifferenzen zwischen verschiedenen (theoretischen) Meßstellen längs des Tauchsensors praktisch nicht beeinflußt wird.

Vorteilhafterweise besteht mindestens eine der Schichten, nämlich Referenz-Material, Festelektrolyt-Material, feuerfester Werkstoff und/oder das korrosionsbeständige Material aus einem aufgespritztem Material; hierdurch wird eine besonders homogene und dichte Schicht erreicht.

Das Referenz-Material besteht zweckmäßigerweise aus einem Metall-Metalloxid und das Festelektrolyt-Material ist vorzugsweise ein Sauerstoffionenleiter. Bevorzugt besteht das Referenz-Material aus Chrom-Chromdioxid und der Festelektrolyt-Überzug aus stabilisiertem Zirkonoxid. Durch die Auswahl dieser Schichtwerkstoffe wird ermöglicht, den Sauerstoffgehalt in Metallschmelzen insbesondere Stahlschmelzen, zu messen.

Bevorzugt weist der Festelektrolyt-Überzug im Bereich seiner Außenseite eine höhere Dichte auf, als im Bereich seiner dem Trägerstift benachbarten Innenseite. Dadurch wird die Stabilität der Messung bei einer kurzen Ansprechzeit, bei einer minimalen Schichtdicke des Festelektrolyt-Überzuges, verbessert.

Vorteilhaft für geringe Abmessungen des Tauchsensors und damit für eine schnelle Ansprechzeit ist es, daß der Trägerstift im Bereich seines freien Endes eine größte Querschnittsfläche zwischen 0,1 mm² und 3 mm², insbesondere zwischen 0,3 mm² und 1,5 mm², bevorzugt von 0,8 mm² aufweist. Die Querschnittsfläche kann dabei eine beliebige Form aufweisen, bevorzugt ist sie kreisförmig oder oval ausgebildet.

Besonders geeignet als Werkstoff für den Trägerstift hat sich Molybdän wegen seiner thermischen Eigenschaften erwiesen.

Um eine zu starke Wärmeableitung durch den Trägerstift zu verhindern, kann der Trägerstift im Bereich des Überzugs aus dem feuerfesten Werktoff wenigstens eine Querschnittsverengung aufweisen.

Weitere Einzelheiten der Erfindung ergeben sich aus der nachfolgenden Erläuterung von Ausführungsbeispielen anhand der Zeichnung. In der Zeichung zeigen
- Figur 1: eine schematische Ausführungsform des erfindungsgemäßen Tauchsensors,
- Figur 2: eine weitere Ausführungsform eines Tauchsensors, bei der gegenüber der Ausführungsform nach Figur 1 das Referenz-Material über eine kürzere Strecke aufgebracht ist,
- Figur 3: eine Anordnung ähnlich der Ausführungsform nach Figur 2 mit einer Querschnittsreduzierung im Endbereich des Trägerstiftes,
- Figur 4: eine Anordnung ähnlich der Ausführungsform nach Figur 2, bei der der vordere Bereich mit einem stabilisierten Zirkondioxid beschichtet ist, während der hintere Bereich eine andere korrosionsbeständige Schicht aufweist, und
- Figur 5: die Darstellung des Porositätsgradienten innerhalb eines Zirkonoxid-Festelektrolyt-Materials.

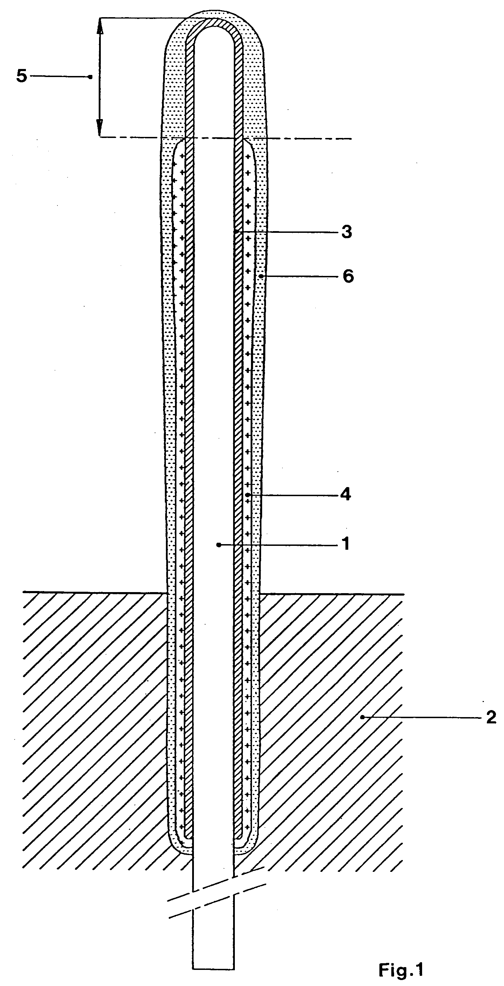

Der Tauchsensor, wie er in Figur 1 dargestellt ist, weist einen Trägerstift 1 auf, der als Ableitelektrode dient und an seinem einen Ende in einer fernerfesten Masse 2 gehaltert ist. Der Trägerstift besitzt einen Querschnitt von 0,8 mm² und besteht aus Molybdän. Auf seiner Oberfläche ist der Trägerstift 1 bis in den feuerfesten Halterungs-Werkstoff 2 hineinreichend mit einem Überzug aus einem Referenz-Material 3 versehen. Bei dem Referenz-Material 3 handelt es sich Chrom-Chromdioxid, das in einer Schichtdicke von etwa 200,um aufgespritzt ist. Auf das Referenz-Material 3 ist ein fernerfester Werkstoff 4 aus Aluminiumoxid über eine solche Länge aufgebracht, daß dieser Überzug einerseits in den feuerfesten Halterungs-Werkstoff 2 hineinreicht und daß er andererseits das Referenz-Material 3 bis zu einem Abstand 5 von etwa 2,5 mm von dem freien Ende des Trägerstiftes 1 überdeckt. Der feuerfeste Werkstoff 4 aus Aluminiumoxid weist in dem außerhalb des feuerfesten Halterungs-Werkstoffes 2 gelegenen Bereich eine Dicke von etwa 200 µm auf. Als dritte Schicht ist ein Festelektrolyt-Material 6 aufgebracht, welches das Referenz-Material 3 und den feuerfesten Werkstoff 4 bedeckt und das bis in den feuerfesten Halterungs-Werkstoff 2 hineinreicht. Das Festelektrolyt-Material 6 besteht aus stabilisiertem Zirkonoxid und weist eine Schichtdicke von etwa 300 µm auf.

Die den Trägerstift 1 bedeckenden Überzüge 3,4 und 6 können mittels Spritzverfahren, beispielsweise mittels Plasmaspritzen oder Flammspritzen, aufgebracht sein, wodurch sich eine besonders gleichmäßige und dichte Beschichtung ergibt. Dabei wird zuerst das Referenz-Material 3 aus Chrom-Chromdioxid auf den Trägerstift 1 aufgebracht, danach wird das nicht eingebettete Ende des Trägerstiftes 1 bis zu einem Abstand von etwa 2,5 mm maskiert und dann auf den unmaskierten Teil des Trägerstiftes 1 der feuerfeste Werkstoff 4 aus Aluminiumoxid aufgebracht. Anschließend wird die Maskierung entfernt und das Festelektrolyt-Material 6 aus stabilisiertem Zirkonoxid aufgespritzt. Nach dem Aufspritzen der Schichten 3,4 und 6 wird der Trägerstift 1 in den feuerfesten Halterungs-Werkstoff 2 eingebracht und dort gehaltert.

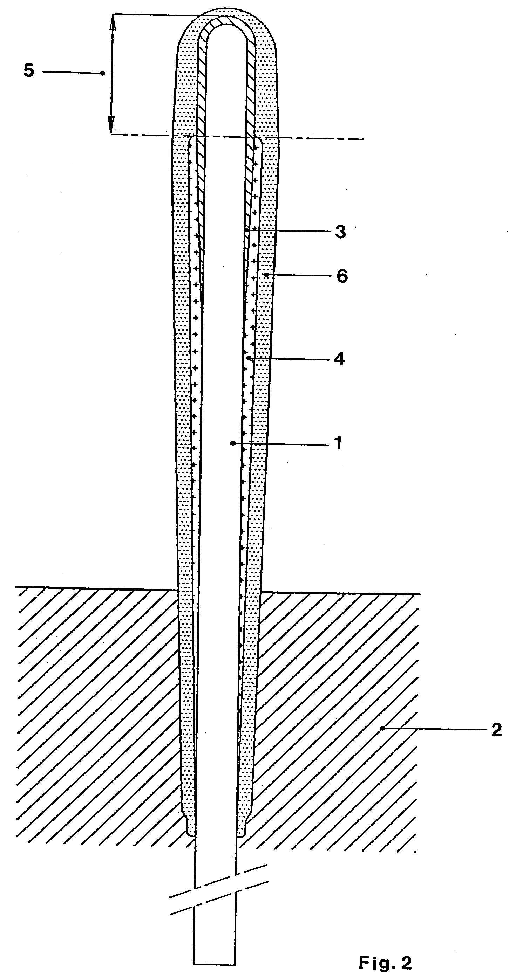

In Figur 2 ist ein Tauchsensor gezeigt, bei dem, im Unterschied zu dem in Figur 1 gezeigten Tauchsensor, das Referenz-Material 3 nicht bis in die feuerfeste Masse 2 hineinreicht, sondern lediglich die Spitze des Trägerstiftes 1 bedeckt und dann unter dem feuerfesten Werkstoff 4 endet. Er läuft dabei im Querschnitt gesehen keilförmig aus. Eine solche Beschichtung wird bevorzugt für den Einsatz des Tauchsensors im Bereich oberhalb 1645°C gewählt, da bei dieser Temperatur das ansonsten ungeschützte Chrom-Chromdioxid schmilzt und dadurch bei einem weiterführenden Überzug des Referenz-Materials 3, bis in den feuerfesten Halterungs-Werkstoff 2 hinein, die mechanische Stabilität des Tauchsensors verloren gehen könnte.

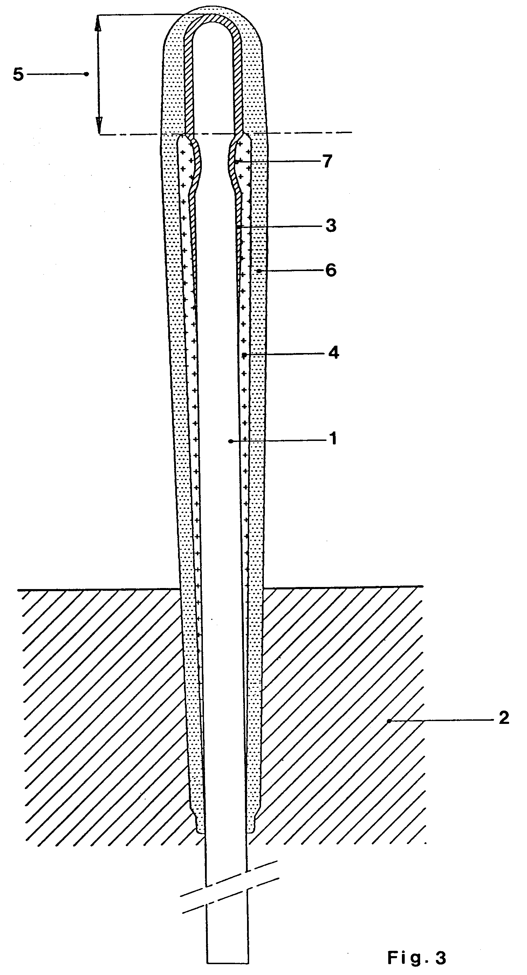

Für eine exakte Messung des EMK-Wertes ist ein elektrochemisches Gleichgewicht zwischen der Metallschmelze und der Tauchsonde notwendig. Ein elektrochemisches Gleichgewicht stellt sich jedoch erst ein, wenn ein thermisches Gleichgewicht zwischen der Tauchsonde und ihrer Umgebung erreicht ist. Um eine Wärmeableitung über den Trägerstift 1 zu verhindern bzw. so gering wie möglich zu halten, ist es notwendig, daß der Trägerstift 1 so dünn wie möglich ist. Eine bestimmte Mindeststärke hat sich jedoch aus Gründen der mechanischen Stabilität als notwendig erwiesen. Optimal ist eine Querschnittsfläche des Trägerstiftes 1 von etwa 0,8 mm², was einem Durchmesser von etwa 1 mm entspricht, wenn der Trägerstift 1 einen kreisförmigen Querschnitt aufweist. Zur weiteren Reduzierung der Wärmeabfuhr ist es gegebenenfalls möglich und von Vorteil, daß der Trägerstift 1 im Bereich des feuerfesten Werkstoffes 4 aus Aluminiumoxid mindestens eine umlaufende Querschnittsverengung 7 aufweist, die die Wärmeableitung über den Trägerstift 1 in Richtung des feuerfesten Halterungs-Werkstoffes 2 herabsetzt. Eine solche Ausführungsform des Tauchsensors ist in Figur 3 dargestellt. Die durch die Querschnittsverengung 7 entstehende Querschnittsfläche beträgt etwa die Hälfte der Querschnittsfläche des Trägerstiftes 1 in dem von dem feuerfesten Werkstoff 4 freien Bereich des Trägerstiftes 1.

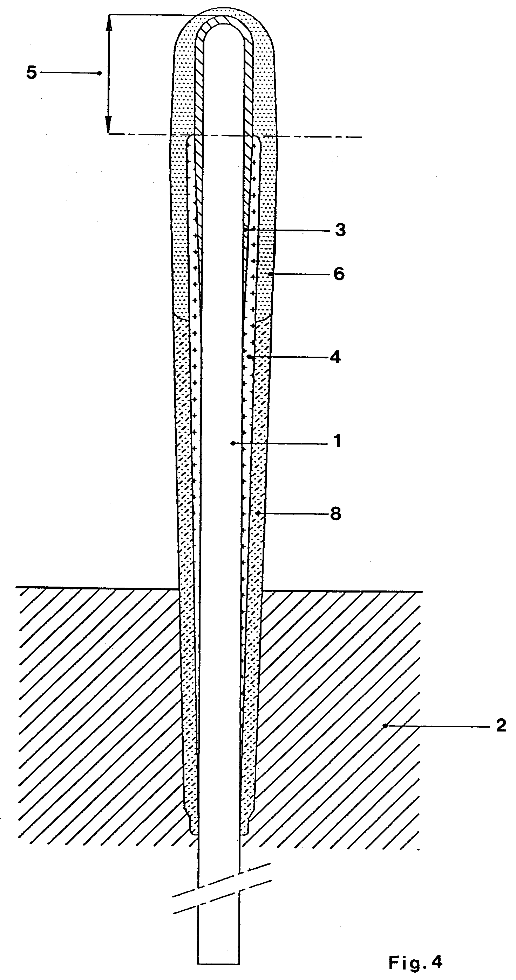

In Figur 4 ist eine weitere Ausführungsform dargestellt. Im Unterschied zu der in Figur 2 dargestellten Ausführung ist der Überzug 6 aus stabilisiertem Zirkondioxid nur im vorderen Bereich des Tauchsensors aufgebracht, so daß das stabilisierte Zirkonoxid lediglich den Endbereich des feuerfesten Werkstoffes 4 bedeckt. An die Schicht 6 aus stabilisiertem Zirkonoxid schließt sich eine Schicht aus einem weiteren korrosionsbeständigem Material 8 an, das den feuerfesten Überzug 4 aus Aluminiumoxid umgibt und bis in den feuerfesten Halterungs-Werkstoff 2 hineinreicht. Dieser korrosionsbeständige Überzug 8 besteht aus zum Beispiel Silikaten, Boriden, Nitriden, Karbiden oder Oxiden.

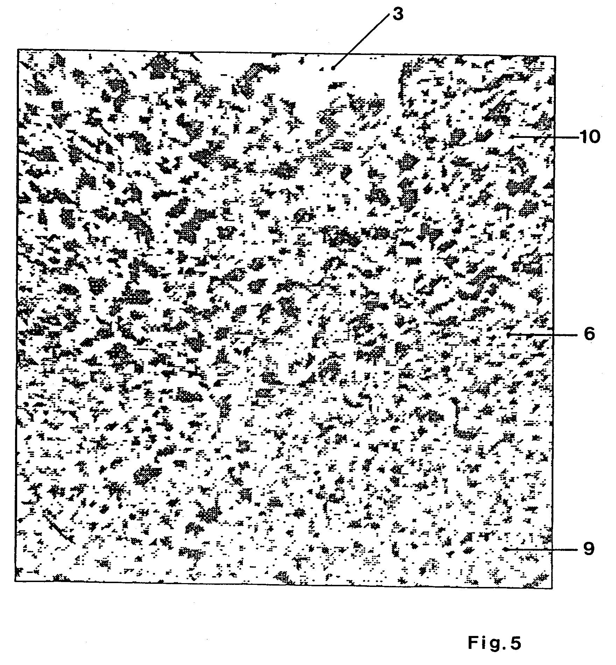

In Figur 5 ist der Porositätsgradient innerhalb des Festelektrolyt-Materials 5 gezeigt. Das Zirkonoxid weist dabei im Bereich seiner Außenseite 9 eine geringere Porosität, also höhere Dichte auf (gekennzeichnet durch die helleren Felder) als im Bereich seiner dem Trägerstift bzw. dem Referenz-Material 3 benachbarten Innenseite 10 (gekennzeichnet durch die dunklen Felder). Durch diesen Porositätsgradienten weist das Zirkonoxid im Bereich seiner Aussenseite 9 eine höhere Korrosionsbeständigkeit auf und es wird im Bereich der dem Trägerstift benachbarten Innenseite 10 ein schneller Ionenaustausch zwischen dem Referenzmaterial 3 und dem Festelektrolyt-Material 6 gewährleistet.

Um ein Zwischenprodukt eines Tauchsensors herzustellen, wird das eine Ende des Trägerstiftes 1 aus Molybdän mit Referenz-Material 3 aus Chrom-Chromdioxid überzogen. Danach wird dieses Ende bis zu einem Abstand 5 von etwa 2,5 mm mit dem mit Referenz-Material 3 überzogenen Ende des Trägerstiftes 1 derart maskiert, daß das dem Ende abgewandte Referenz-Material 3 unmaskiert verbleibt. Dieser unmaskierte Teil des Trägerstiftes 1 wird mit einem feuerfesten Werkstoff 4 aus Aluminiumoxid überzogen. Anschließend wird die Maskierung entfernt und auf das Referenz-Material 3 ein Überzug aus dem Festelektrolyt-Material 6 aus stabilisiertem Zirkonoxid so aufgebracht, daß dieser Überzug mindestens das dem Ende des Trägerstiftes 1 benachbarte Ende des Überzugs aus dem fernerfesten Werkstoff 4 überdeckt.

## Patentansprüche

1. Tauchsensor für Metallschmelzen, der einen elektrisch leitenden Trägerstift aufweist, dessen eines Ende in einem feuerfesten Halterungs-Werkstoff gehaltert ist und dessen anderes, freies Ende einen ersten Überzug aus einem Referenz-Material und darauf einen zweiten Überzug aus einem Festelektrolyt-Material aufweist, wobei der Trägerstift im Bereich seines gehalterten Endes einen dritten Überzug aus einem feuerfesten Werkstoff aufweist, der einerseits in den Halterungs-Werkstoff hineinreicht und der sich andererseits bis zum Festelektrolyt-Material erstreckt, dadurch gekennzeichnet, daß das Festelektrolyt-Material (6) mindestens den Endbereich des ihm benachbarten feuerfesten Werkstoffes (4) überdeckt.

2. Tauchsensor nach Anspruch 1, dadurch gekennzeichnet, daß der feuerfeste Werkstoff (4) mit einem gegenüber der Metallschmelze korrosionsbeständigen Material (8) überzogen ist.

3. Tauchsensor nach Anspruch 2, dadurch gekennzeichnet, daß das korrosionsbeständige Material (8) ein Festelektrolyt-Werkstoff ist.

4. Tauchsensor nach Anspruch 3, dadurch gekennzeichnet, daß das korrosionsbeständige Material (8) dasselbe Material ist wie der Festelektrolyt-Überzug (6).

5. Tauchsensor nach einem der Ansprüche 1 bis 4, dadurch gekennzeichnet, daß der feuerfeste Werkstoff (4) in einem Abstand von l bis 6 mm vom freien Ende des Trägerstiftes (1) beginnt.

6. Tauchsensor nach Anspruch 5, dadurch gekennzeichnet, daß der Abstand 1,5 bis 3,5 mm beträgt.

7. Tauchsensor nach Anspruch 6, dadurch gekennzeichnet, daß der Abstand etwa 2,5 mm beträgt.

8. Tauchsensor nach einem der Ansprüche 1 bis 7, dadurch gekennzeichnet, daß das Referenz-Material (3) und das Festelektrolyt-Material (6) aus aufgespritztem Material besteht.

9. Tauchsensor nach einem der Ansprüche 1 bis 8, dadurch gekennzeichnet, daß der feuerfeste Werkstoff (4) aus einem aufgespritztem Material besteht.

10. Tauchsensor nach einem der Ansprüche 2 bis 9, dadurch gekennzeichnet, daß das korrosionsbeständige Material (8) aus einem aufgespritztem Material besteht.

11. Tauchsensor nach einem der Ansprüche 1 bis 10, dadurch gekennzeichnet, daß das Referenz-Material (3) aus einem Metall-Metalloxid besteht und daß das Festelektrolyt-Material (6) ein Sauerstoffionenleiter ist.

12. Tauchsensor nach Anspruch 11, dadurch gekennzeichnet, daß das Referenz-Material (3) aus Chrom-Chromdioxid und der Festelektrolyt-Überzug (6) aus stabilisiertem Zirkonoxid besteht.

13. Tauchsensor nach einem der Ansprüche 1 bis 12, dadurch gekennzeichnet, daß der Festelektrolyt-Überzug (6) im Bereich seiner Außenseite (9) eine höhere Dichte aufweist, als im Bereich seiner dem Trägerstift (1) benachbarten Innenseite (10).

14. Tauchsensor nach einem der Ansprüche 1 bis 13, dadurch gekennzeichnet, daß der Trägerstift (1) im Bereich seines freien Endes eine größte Querschnittsfläche zwischen 0,1 mm² und 3 mm² aufweist.

15. Tauchsensor nach Anspruch 14, dadurch gekennzeichnet, daß die Querschnittsfläche zwischen 0,3 mm² und 1,5 mm² liegt.

16. Tauchsensor nach Anspruch 15, dadurch gekennzeichnet, daß die Querschnittsfläche etwa 0,8 mm² beträgt.

17. Tauchsensor nach einem der Ansprüche 1 bis 16, dadurch gekennzeichnet, daß der Trägerstift (1) aus Molybdän besteht.

18. Tauchsensor nach einem der Ansprüche 1 bis 17, dadurch gekennzeichnet, daß der Trägerstift (1) im Bereich des Überzugs aus dem feuerfesten Werkstoff (4) wenigstens eine Querschnittsverengung (7) aufweist.

19. Verfahren zur Herstellung eines Zwischenproduktes eines Tauchsensors, dadurch gekennzeichnet, daß auf einem Trägerstift (1) auf dessen eines Ende ein Überzug aus einem Referenz-Material (3) aufgebracht wird, daß danach dieses Ende derart maskiert wird, daß das Referenz-Material (3), das dem Ende abgewandt ist, unmaskiert verbleibt und auf dem unmaskierten Teil des Trägerstiftes (1) ein Überzug aus feuerfestem Werkstoff 4 aufgebracht wird, und daß anschließend auf das Referenz-Material (3) ein Überzug aus einem Festelektrolyt-Material (6) nach Entfernung der Maskierung so aufgebracht wird, daß dieser Überzug das dem Ende des Trägerstiftes (1) benachbarte Ende des Überzugs aus feuerfestem Werkstoff (4) überdeckt.
